# EUROPEAN PATENT APPLICATION

(11) **EP 2 272 979 A1**
(43) Date of publication of application: **12.01.2011**
(21) Application number: 09305633.1
(22) Date of filing: 30.06.2009
(51) Int. Cl.: C12Q 1/68

(54) **Method for testing a subject thought to be predisposed to having cancer**

(71) Applicant: Centre National de la Recherche Scientifique (CNRS), 75016 Paris (FR)
(72) Inventor: Roux, Pierre, 34980 Saint Gely du Fesc (FR); Gadea, Gilles, 34090 Montpellier (FR); Vinot, Stéphanie, 34090 Montpellier (FR); Anguille, Chritelle, 34090 Montpellier (FR); Bourdon, Jean-Christophe, Dundee, DD2 1QD (GB); Fernandes, Kenneth, Dundee, DD5 2ST (GB)
(74) Representative: Warcoin, Jacques

(57) **Abstract**

The present invention concerns a method of testing a subject thought to be predisposed to having cancer which comprises the step of i) analyzing a biological sample from said subject for detecting the presence, the expression level or the absence of a p53 isoform selected in the group consisting in Δ133p53, Δ133p53γ and Δ133p53β, the presence of said p53 isoform being indicative of a cancer.

SEQUENCE LISTING

<110>  Centre National de la Recherche Scientifique (CNRS)

<120>  Method for testing a subject thought to be predisposed to having cancer

<130>  68114

<160>  11

<170>  PatentIn version 3.3

<210>  1
<211>  209
<212>  PRT
<213>  homo sapiens

<220>
<221>  misc_feature
<223>  Homo sapiens tumor protein delta133p53beta (Genbank NM
       001126116.1)

<400>  1

Met Phe Cys Gln Leu Ala Lys Thr Cys Pro Val Gln Leu Trp Val Asp
1               5                   10                  15

Ser Thr Pro Pro Pro Gly Thr Arg Val Arg Ala Met Ala Ile Tyr Lys
            20                  25                  30

Gln Ser Gln His Met Thr Glu Val Val Arg Arg Cys Pro His His Glu
        35                  40                  45

Arg Cys Ser Asp Ser Asp Gly Leu Ala Pro Pro Gln His Leu Ile Arg
    50                  55                  60

Val Glu Gly Asn Leu Arg Val Glu Tyr Leu Asp Asp Arg Asn Thr Phe
65                  70                  75                  80

Arg His Ser Val Val Val Pro Tyr Glu Pro Pro Glu Val Gly Ser Asp
                85                  90                  95

Cys Thr Thr Ile His Tyr Asn Tyr Met Cys Asn Ser Ser Cys Met Gly
            100                 105                 110

Gly Met Asn Arg Arg Pro Ile Leu Thr Ile Ile Thr Leu Glu Asp Ser
        115                 120                 125

Ser Gly Asn Leu Leu Gly Arg Asn Ser Phe Glu Val Arg Val Cys Ala
    130                 135                 140

Cys Pro Gly Arg Asp Arg Arg Thr Glu Glu Glu Asn Leu Arg Lys Lys
145                 150                 155                 160

Gly Glu Pro His His Glu Leu Pro Pro Gly Ser Thr Lys Arg Ala Leu
                165                 170                 175

Pro Asn Asn Thr Ser Ser Ser Pro Gln Pro Lys Lys Lys Pro Leu Asp
            180                 185                 190

Gly Glu Tyr Phe Thr Leu Gln Asp Gln Thr Ser Phe Gln Lys Glu Asn
        195                 200                 205

Cys

<210>  2
<211>  2404
<212>  DNA
<213>  homo sapiens

<220>
<221>  misc_feature
<223>  Homo sapiens tumor protein delta133p53beta (Genbank NM
       001126116.1)

<400>  2
tgaggccagg agatggaggc tgcagtgagc tgtgatcaca ccactgtgct ccagcctgag      60

tgacagagca agaccctatc tcaaaaaaaa aaaaaaaaaa gaaaagctcc tgaggtgtag     120

acgccaactc tctctagctc gctagtgggt tgcaggaggt gcttacgcat gtttgtttct     180

ttgctgccgt cttccagttg ctttatctgt tcacttgtgc cctgactttc aactctgtct     240

ccttcctctt cctacagtac tcccctgccc tcaacaagat gttttgccaa ctggccaaga     300

cctgccctgt gcagctgtgg gttgattcca cacccccgcc cggcacccgc gtccgcgcca     360

tggccatcta caagcagtca cagcacatga cggaggttgt gaggcgctgc ccccaccatg     420

agcgctgctc agatagcgat ggtctggccc ctcctcagca tcttatccga gtggaaggaa     480

atttgcgtgt ggagtatttg gatgacagaa acacttttcg acatagtgtg gtggtgccct     540

atgagccgcc tgaggttggc tctgactgta ccaccatcca ctacaactac atgtgtaaca     600

gttcctgcat gggcggcatg aaccggaggc ccatcctcac catcatcaca ctggaagact     660

ccagtggtaa tctactggga cggaacagct ttgaggtgcg tgtttgtgcc tgtcctggga     720

gagaccggcg cacagaggaa gagaatctcc gcaagaaagg ggagcctcac cacgagctgc     780

ccccagggag cactaagcga gcactgccca acaacaccag ctcctctccc cagccaaaga     840

agaaaccact ggatggagaa tatttcaccc ttcaggacca gaccagcttt caaaaagaaa     900

attgttaaag agagcatgaa aatggttcta tgactttgcc tgatacagat gctacttgac     960

ttacgatggt gttacttcct gataaactcg tcgtaagttg aaaatattat ccgtgggcgt    1020

gagcgcttcg agatgttccg agagctgaat gaggccttgg aactcaagga tgcccaggct    1080

gggaaggagc caggggggag cagggctcac tccagccacc tgaagtccaa aaagggtcag    1140

tctacctccc gccataaaaa actcatgttc aagacagaag ggcctgactc agactgacat    1200

tctccacttc ttgttcccca ctgacagcct cccaccccca tctctccctc ccctgccatt    1260

ttgggttttg ggtctttgaa cccttgcttg caataggtgt gcgtcagaag cacccaggac    1320

ttccatttgc tttgtcccgg ggctccactg aacaagttgg cctgcactgg tgttttgttg 1380

tggggaggag gatggggagt aggacatacc agcttagatt ttaaggtttt tactgtgagg 1440

gatgtttggg agatgtaaga aatgttcttg cagttaaggg ttagtttaca atcagccaca 1500

ttctaggtag gggcccactt caccgtacta accagggaag ctgtccctca ctgttgaatt 1560

ttctctaact tcaaggccca tatctgtgaa atgctggcat ttgcacctac ctcacagagt 1620

gcattgtgag ggttaatgaa ataatgtaca tctggccttg aaaccacctt ttattacatg 1680

gggtctagaa cttgaccccc ttgagggtgc ttgttccctc tccctgttgg tcggtgggtt 1740

ggtagtttct acagttgggc agctggttag gtagagggag ttgtcaagtc tctgctggcc 1800

cagccaaacc ctgtctgaca acctcttggt gaaccttagt acctaaaagg aaatctcacc 1860

ccatcccaca ccctggagga tttcatctct tgtatatgat gatctggatc caccaagact 1920

tgttttatgc tcagggtcaa tttctttttt cttttttttt tttttttttc tttttctttg 1980

agactgggtc tcgctttgtt gcccaggctg gagtggagtg gcgtgatctt ggcttactgc 2040

agcctttgcc tccccggctc gagcagtcct gcctcagcct ccggagtagc tgggaccaca 2100

ggttcatgcc accatggcca gccaactttt gcatgttttg tagagatggg gtctcacagt 2160

gttgcccagg ctggtctcaa actcctgggc tcaggcgatc cacctgtctc agcctcccag 2220

agtgctggga ttacaattgt gagccaccac gtccagctgg aagggtcaac atcttttaca 2280

ttctgcaagc acatctgcat tttcacccca cccttcccct ccttctccct ttttatatcc 2340

catttttata tcgatctctt attttacaat aaaactttgc tgccacctgt gtgtctgagg 2400

ggtg 2404

<210> 3
<211> 261
<212> PRT
<213> homo sapiens

<220>
<221> misc_feature
<223> Homo sapiens tumor protein delta133p53 (Genbank NM 001126115.1)

<400> 3

Met Phe Cys Gln Leu Ala Lys Thr Cys Pro Val Gln Leu Trp Val Asp
1               5                   10                  15

Ser Thr Pro Pro Pro Gly Thr Arg Val Arg Ala Met Ala Ile Tyr Lys
            20                  25                  30

Gln Ser Gln His Met Thr Glu Val Val Arg Arg Cys Pro His His Glu
        35                  40                  45

Arg Cys Ser Asp Ser Asp Gly Leu Ala Pro Pro Gln His Leu Ile Arg
    50                  55                  60

Val Glu Gly Asn Leu Arg Val Glu Tyr Leu Asp Asp Arg Asn Thr Phe
65                  70                  75                  80

Arg His Ser Val Val Val Pro Tyr Glu Pro Pro Glu Val Gly Ser Asp
                85                  90                  95

Cys Thr Thr Ile His Tyr Asn Tyr Met Cys Asn Ser Ser Cys Met Gly
            100                 105                 110

Gly Met Asn Arg Arg Pro Ile Leu Thr Ile Ile Thr Leu Glu Asp Ser
        115                 120                 125

Ser Gly Asn Leu Leu Gly Arg Asn Ser Phe Glu Val Arg Val Cys Ala
    130                 135                 140

Cys Pro Gly Arg Asp Arg Arg Thr Glu Glu Glu Asn Leu Arg Lys Lys
145                 150                 155                 160

Gly Glu Pro His His Glu Leu Pro Pro Gly Ser Thr Lys Arg Ala Leu
                165                 170                 175

Pro Asn Asn Thr Ser Ser Ser Pro Gln Pro Lys Lys Lys Pro Leu Asp
            180                 185                 190

Gly Glu Tyr Phe Thr Leu Gln Ile Arg Gly Arg Glu Arg Phe Glu Met
        195                 200                 205

Phe Arg Glu Leu Asn Glu Ala Leu Glu Leu Lys Asp Ala Gln Ala Gly
    210                 215                 220

Lys Glu Pro Gly Gly Ser Arg Ala His Ser Ser His Leu Lys Ser Lys
225                 230                 235                 240

Lys Gly Gln Ser Thr Ser Arg His Lys Lys Leu Met Phe Lys Thr Glu
                245                 250                 255

Gly Pro Asp Ser Asp
            260

<210>  4
<211>  2271
<212>  DNA
<213>  homo sapiens

<220>
<221>  misc_feature
<223>  Homo sapiens tumor protein delta133p53 (Genbank NM 001126115.1)

<400>  4
tgaggccagg agatggaggc tgcagtgagc tgtgatcaca ccactgtgct ccagcctgag      60

tgacagagca agaccctatc tcaaaaaaaa aaaaaaaaaa gaaaagctcc tgaggtgtag     120

acgccaactc tctctagctc gctagtgggt tgcaggaggt gcttacgcat gtttgtttct     180

ttgctgccgt cttccagttg ctttatctgt tcacttgtgc cctgactttc aactctgtct     240

ccttcctctt cctacagtac tcccctgccc tcaacaagat gttttgccaa ctggccaaga 300
cctgccctgt gcagctgtgg gttgattcca cacccccgcc cggcacccgc gtccgcgcca 360
tggccatcta caagcagtca cagcacatga cggaggttgt gaggcgctgc ccccaccatg 420
agcgctgctc agatagcgat ggtctggccc ctcctcagca tcttatccga gtggaaggaa 480
atttgcgtgt ggagtatttg gatgacagaa acacttttcg acatagtgtg gtggtgccct 540
atgagccgcc tgaggttggc tctgactgta ccaccatcca ctacaactac atgtgtaaca 600
gttcctgcat gggcggcatg aaccggaggc ccatcctcac catcatcaca ctggaagact 660
ccagtggtaa tctactggga cggaacagct ttgaggtgcg tgtttgtgcc tgtcctggga 720
gagaccggcg cacagaggaa gagaatctcc gcaagaaagg ggagcctcac cacgagctgc 780
ccccagggag cactaagcga gcactgccca acaacaccag ctcctctccc cagccaaaga 840
agaaaccact ggatggagaa tatttcaccc ttcagatccg tgggcgtgag cgcttcgaga 900
tgttccgaga gctgaatgag gccttggaac tcaaggatgc ccaggctggg aaggagccag 960
gggggagcag ggctcactcc agccacctga agtccaaaaa gggtcagtct acctcccgcc 1020
ataaaaaact catgttcaag acagaagggc ctgactcaga ctgacattct ccacttcttg 1080
ttccccactg acagcctccc acccccatct ctccctcccc tgccattttg ggttttgggt 1140
ctttgaaccc ttgcttgcaa taggtgtgcg tcagaagcac ccaggacttc catttgcttt 1200
gtcccggggc tccactgaac aagttggcct gcactggtgt tttgttgtgg ggaggaggat 1260
ggggagtagg acataccagc ttagatttta aggtttttac tgtgagggat gtttgggaga 1320
tgtaagaaat gttcttgcag ttaagggtta gtttacaatc agccacattc taggtagggg 1380
cccacttcac cgtactaacc agggaagctg tccctcactg ttgaattttc tctaacttca 1440
aggcccatat ctgtgaaatg ctggcatttg cacctacctc acagagtgca ttgtgagggt 1500
taatgaaata atgtacatct ggccttgaaa ccacctttta ttacatgggg tctagaactt 1560
gacccccttg agggtgcttg ttccctctcc ctgttggtcg gtgggttggt agtttctaca 1620
gttgggcagc tggttaggta gagggagttg tcaagtctct gctggcccag ccaaaccctg 1680
tctgacaacc tcttggtgaa ccttagtacc taaaaggaaa tctcacccca tcccacaccc 1740
tggaggattt catctcttgt atatgatgat ctggatccac caagacttgt tttatgctca 1800
gggtcaattt ctttttttct tttttttttt tttttctttt ttctttgaga ctgggtctcg 1860
ctttgttgcc caggctggag tggagtggcg tgatcttggc ttactgcagc ctttgcctcc 1920
ccggctcgag cagtcctgcc tcagcctccg gagtagctgg gaccacaggt tcatgccacc 1980
atggccagcc aacttttgca tgttttgtag agatggggtc tcacagtgtt gcccaggctg 2040
gtctcaaact cctgggctca ggcgatccac ctgtctcagc ctcccagagt gctgggatta 2100
caattgtgag ccaccacgtc cagctggaag ggtcaacatc ttttacattc tgcaagcaca 2160
tctgcatttt caccccaccc ttcccctcct tctccctttt tatatcccat ttttatatcg 2220
atctcttatt ttacaataaa actttgctgc cacctgtgtg tctgaggggt g 2271

<210>  5
<211>  214
<212>  PRT
<213>  homo sapiens


<220>
<221>  misc_feature
<223>  Homo sapiens tumor protein delta133p53gamma (Genbank NM
       001126117.1)

<400>  5

Met Phe Cys Gln Leu Ala Lys Thr Cys Pro Val Gln Leu Trp Val Asp
1               5                   10                  15


Ser Thr Pro Pro Pro Gly Thr Arg Val Arg Ala Met Ala Ile Tyr Lys
            20                  25                  30


Gln Ser Gln His Met Thr Glu Val Val Arg Arg Cys Pro His His Glu
        35                  40                  45


Arg Cys Ser Asp Ser Asp Gly Leu Ala Pro Pro Gln His Leu Ile Arg
    50                  55                  60


Val Glu Gly Asn Leu Arg Val Glu Tyr Leu Asp Asp Arg Asn Thr Phe
65                  70                  75                  80


Arg His Ser Val Val Val Pro Tyr Glu Pro Pro Glu Val Gly Ser Asp
                85                  90                  95


Cys Thr Thr Ile His Tyr Asn Tyr Met Cys Asn Ser Ser Cys Met Gly
            100                 105                 110


Gly Met Asn Arg Arg Pro Ile Leu Thr Ile Ile Thr Leu Glu Asp Ser
        115                 120                 125


Ser Gly Asn Leu Leu Gly Arg Asn Ser Phe Glu Val Arg Val Cys Ala
    130                 135                 140


Cys Pro Gly Arg Asp Arg Arg Thr Glu Glu Glu Asn Leu Arg Lys Lys
145                 150                 155                 160


Gly Glu Pro His His Glu Leu Pro Pro Gly Ser Thr Lys Arg Ala Leu
                165                 170                 175


Pro Asn Asn Thr Ser Ser Ser Pro Gln Pro Lys Lys Lys Pro Leu Asp
            180                 185                 190


Gly Glu Tyr Phe Thr Leu Gln Met Leu Leu Asp Leu Arg Trp Cys Tyr
        195                 200                 205


Phe Leu Ile Asn Ser Ser
    210

<210>  6
<211>  2331
<212>  DNA
<213>  homo sapiens

<220>
<221>  misc_feature
<223>  Homo sapiens tumor protein p53, delta133p53gamma (Genbank NM
       001126117.1)

<400>  6
tgaggccagg agatggaggc tgcagtgagc tgtgatcaca ccactgtgct ccagcctgag      60

tgacagagca agaccctatc tcaaaaaaaa aaaaaaaaaa gaaaagctcc tgaggtgtag     120

acgccaactc tctctagctc gctagtgggt tgcaggaggt gcttacgcat gtttgtttct     180

ttgctgccgt cttccagttg ctttatctgt tcacttgtgc cctgactttc aactctgtct     240

ccttcctctt cctacagtac tcccctgccc tcaacaagat gttttgccaa ctggccaaga     300

cctgccctgt gcagctgtgg gttgattcca caccccgccc cggcacccgc gtccgcgcca     360

tggccatcta caagcagtca cagcacatga cggaggttgt gaggcgctgc ccccaccatg     420

agcgctgctc agatagcgat ggtctggccc ctcctcagca tcttatccga gtggaaggaa     480

atttgcgtgt ggagtatttg gatgacagaa acacttttcg acatagtgtg gtggtgccct     540

atgagccgcc tgaggttggc tctgactgta ccaccatcca ctacaactac atgtgtaaca     600

gttcctgcat gggcggcatg aaccggaggc ccatcctcac catcatcaca ctggaagact     660

ccagtggtaa tctactggga cggaacagct ttgaggtgcg tgtttgtgcc tgtcctggga     720

gagaccggcg cacagaggaa gagaatctcc gcaagaaagg ggagcctcac cacgagctgc     780

ccccagggag cactaagcga gcactgccca acaacaccag ctcctctccc cagccaaaga     840

agaaaccact ggatggagaa tatttcaccc ttcagatgct acttgactta cgatggtgtt     900

acttcctgat aaactcgtcg taagttgaaa atattatccg tgggcgtgag cgcttcgaga     960

tgttccgaga gctgaatgag gccttggaac tcaaggatgc ccaggctggg aaggagccag    1020

gggggagcag ggctcactcc agccacctga agtccaaaaa gggtcagtct acctcccgcc    1080

ataaaaaact catgttcaag acagaagggc ctgactcaga ctgacattct ccacttcttg    1140

ttccccactg acagcctccc acccccatct ctccctcccc tgccattttg ggttttgggt    1200

ctttgaaccc ttgcttgcaa taggtgtgcg tcagaagcac ccaggacttc catttgcttt    1260

gtcccggggc tccactgaac aagttggcct gcactggtgt tttgttgtgg ggaggaggat    1320

ggggagtagg acataccagc ttagatttta aggtttttac tgtgagggat gtttgggaga    1380

tgtaagaaat gttcttgcag ttaagggtta gtttacaatc agccacattc taggtagggg    1440

cccacttcac cgtactaacc agggaagctg tccctcactg ttgaattttc tctaacttca    1500

aggcccatat ctgtgaaatg ctggcatttg cacctacctc acagagtgca ttgtgagggt    1560

taatgaaata atgtacatct ggccttgaaa ccacctttta ttacatgggg tctagaactt    1620

gacccccttg agggtgcttg ttccctctcc ctgttggtcg gtgggttggt agtttctaca    1680

gttgggcagc tggttaggta gagggagttg tcaagtctct gctggcccag ccaaaccctg    1740

tctgacaacc tcttggtgaa ccttagtacc taaaaggaaa tctcacccca tcccacaccc 1800

tggaggattt catctcttgt atatgatgat ctggatccac caagacttgt tttatgctca 1860

gggtcaattt cttttttctt tttttttttt tttttctttt ttctttgaga ctgggtctcg 1920

ctttgttgcc caggctggag tggagtggcg tgatcttggc ttactgcagc ctttgcctcc 1980

ccggctcgag cagtcctgcc tcagcctccg gagtagctgg gaccacaggt tcatgccacc 2040

atggccagcc aacttttgca tgttttgtag agatggggtc tcacagtgtt gcccaggctg 2100

gtctcaaact cctgggctca ggcgatccac ctgtctcagc ctcccagagt gctgggatta 2160

caattgtgag ccaccacgtc cagctggaag ggtcaacatc ttttacattc tgcaagcaca 2220

tctgcatttt caccccaccc ttcccctcct tctccctttt tatatcccat ttttatatcg 2280

atctcttatt ttacaataaa actttgctgc cacctgtgtg tctgaggggt g 2331

<210> 7
<211> 393
<212> PRT
<213> homo sapiens

<220>
<221> misc_feature
<223> Homo sapiens tumor protein p53 (Genbank NM 000546.4)

<400> 7

<210>  8
<211>  2586
<212>  DNA
<213>  homo sapiens

<220>
<221>  misc_feature
<223>  Homo sapiens tumor protein p53 (Genbank NM 000546.4)

<400>  8
   gattggggtt ttcccctccc atgtgctcaa gactggcgct aaaagttttg agcttctcaa      60
   aagtctagag ccaccgtcca gggagcaggt agctgctggg ctccggggac actttgcgtt     120
   cgggctggga gcgtgctttc cacgacggtg acacgcttcc ctggattggc agccagactg     180
   ccttccgggt cactgccatg gaggagccgc agtcagatcc tagcgtcgag ccccctctga     240
   gtcaggaaac attttcagac ctatggaaac tacttcctga aaacaacgtt ctgtccccct     300
   tgccgtccca agcaatggat gatttgatgc tgtccccgga cgatattgaa caatggttca     360
   ctgaagaccc aggtccagat gaagctccca gaatgccaga ggctgctccc cccgtggccc     420
   ctgcaccagc agctcctaca ccggcggccc ctgcaccagc cccctcctgg cccctgtcat     480
   cttctgtccc ttcccagaaa acctaccagg gcagctacgg tttccgtctg ggcttcttgc     540
   attctgggac agccaagtct gtgacttgca cgtactcccc tgccctcaac aagatgtttt     600
   gccaactggc caagacctgc cctgtgcagc tgtgggttga ttccacaccc ccgcccggca     660
   cccgcgtccg cgccatggcc atctacaagc agtcacagca catgacggag gttgtgaggc     720
   gctgccccca ccatgagcgc tgctcagata gcgatggtct ggcccctcct cagcatctta     780
   tccgagtgga aggaaatttg cgtgtggagt atttggatga cagaaacact tttcgacata     840
   gtgtggtggt gccctatgag ccgcctgagg ttggctctga ctgtaccacc atccactaca     900
   actacatgtg taacagttcc tgcatgggcg gcatgaaccg gaggcccatc ctcaccatca     960
   tcacactgga agactccagt ggtaatctac tgggacggaa cagctttgag gtgcgtgttt    1020
   gtgcctgtcc tgggagagac cggcgcacag aggaagagaa tctccgcaag aaaggggagc    1080
   ctcaccacga gctgccccca gggagcacta agcgagcact gcccaacaac accagctcct    1140
   ctccccagcc aaagaagaaa ccactggatg gagaatattt cacccttcag atccgtgggc    1200
   gtgagcgctt cgagatgttc cgagagctga atgaggcctt ggaactcaag gatgcccagg    1260
   ctgggaagga gccagggggg agcagggctc actccagcca cctgaagtcc aaaaagggtc    1320
   agtctacctc ccgccataaa aaactcatgt tcaagacaga agggcctgac tcagactgac    1380
   attctccact tcttgttccc cactgacagc ctcccacccc catctctccc tcccctgcca    1440
   ttttgggttt tgggtctttg aacccttgct tgcaataggt gtgcgtcaga agcacccagg    1500
   acttccattt gctttgtccc ggggctccac tgaacaagtt ggcctgcact ggtgttttgt    1560
   tgtggggagg aggatgggga gtaggacata ccagcttaga ttttaaggtt tttactgtga    1620
   gggatgtttg ggagatgtaa gaaatgttct tgcagttaag ggttagttta caatcagcca    1680
   cattctaggt aggggcccac ttcaccgtac taaccaggga agctgtccct cactgttgaa    1740
   ttttctctaa cttcaaggcc catatctgtg aaatgctggc atttgcacct acctcacaga    1800
   gtgcattgtg agggttaatg aaataatgta catctggcct tgaaaccacc ttttattaca    1860
   tggggtctag aacttgaccc ccttgagggt gcttgttccc tctccctgtt ggtcggtggg 1920
   ttggtagttt ctacagttgg gcagctggtt aggtagaggg agttgtcaag tctctgctgg 1980
   cccagccaaa ccctgtctga caacctcttg gtgaacctta gtacctaaaa ggaaatctca 2040
   ccccatccca caccctggag gatttcatct cttgtatatg atgatctgga tccaccaaga 2100
   cttgttttat gctcagggtc aatttctttt ttcttttttt tttttttttt tctttttctt 2160
   tgagactggg tctcgctttg ttgcccaggc tggagtggag tggcgtgatc ttggcttact 2220
   gcagcctttg cctccccggc tcgagcagtc ctgcctcagc ctccggagta gctgggacca 2280
   caggttcatg ccaccatggc cagccaactt ttgcatgttt tgtagagatg gggtctcaca 2340
   gtgttgccca ggctggtctc aaactcctgg gctcaggcga tccacctgtc tcagcctccc 2400
   agagtgctgg gattacaatt gtgagccacc acgtccagct ggaagggtca acatctttta 2460
   cattctgcaa gcacatctgc attttcaccc cacccttccc ctccttctcc ctttttatat 2520
   cccattttta tatcgatctc ttattttaca ataaaacttt gctgccacct gtgtgtctga 2580
   ggggtg 2586

<210> 9
<211> 26
<212> DNA
<213> Artificial

<220>
<223> Forward primer DNp53-228F for the detection of delta133p53,
delta133p53beta and delta133p53gamma

<400> 9
actctgtctc cttcctcttc ctacag 26

<210> 10
<211> 21
<212> DNA
<213> Artificial

<220>
<223> Reverse primer JWR for the detection of delta133p53,
delta133p53beta and delta133p53gamma

<400> 10
gtgtggaatc aacccacagc t 21

<210> 11
<211> 28
<212> DNA
<213> Artificial

<220>
<223> p53 probe for the detection of delta133p53, delta133p53beta and
delta133p53gamma

<400> 11
tcccctgccc tcaacaagat gttttgcc 28

## Description

### Field of the Invention

The present invention relates to a method of testing a subject thought to be predisposed to having cancer, preferably a breast cancer or a colon cancer.

### Background of the invention

Despite efforts to improve treatment and management of cancer patients, survival in cancer patients has not improved over the past two decades for many cancer types. A critical event during tumorigenesis is the conversion of a primary, localised, tumour into an invasive metastasis. Most of the patients having metastasis died of it. Only 35% of the newly detected patients do not present metastasis.

The invasive process is a consequence of morphological modifications of cells which begins with the loss of adhesiveness due to the disruption of epithelial junctions. Then, cells acquire migration ability through remodelling of the actin cytoskeleton, which involves Rho family of small GTPases. Tumour cells can employ two modes of migration: mesenchymal migration which requires integrin-mediated adhesion dynamics and surface proteases to degrade the extracellular matrix (ECM) or amoeboid migration which is more effective and does not require ECM digestion or integrin-mediated adhesion but involves the RhoA/ROCK pathway. The tumour suppressor p53 prevents cancer progression to invasiveness by modulating Rho GTPase-mediated cell motility.

Optimal therapy will be based on a combination of diagnostic and prognostic information. An accurate and reproducible diagnostic test is needed to provide a prognostic assessment that will provide specific information regarding survival. In breast cancer, the clinical and biological variables commonly used to predict the survival of primary chirurgical treatments are regional lymph nodes invasion, histological grade, and hormone receptor expression. All these parameters are well recognized prognostic and predictive factors.

Nevertheless, these variables do not enable to establish a specific and complete survival prognostic. In fact, there is an important heterogeneity between patients suffering from the same breast cancer type.

Thus, there is an existing need to provide further biomarkers that will strengthen the prediction of survival in breast cancer patients.

Biomarkers are useful in biology to distinguish between a normally medical state and a pathological state, or to evaluate the progression of pathology. Technologies permitting the early detection of cancer thanks to biomarkers should be very benific in cancer search and in clinical cares.

The interest of a tumoral marker is to establish, to evalutate and to validate molecular classification of the human tumours, thus permitting, from the diagnostic:
- to better evaluate the infraclinic metastatic potential of cancers, thus avoiding an overtreatment of the patients presenting a low risk and thus permitting to isolate patients presenting a high risk of evolution;
- to switch from a group evaluation to a personal evaluation thus rendering possible personalized therapies;
- to direct patients presenting a high risk of evolution and non or almost non sensitive to conventional treatments to therapeutical innovations; and
- to permit to evaluate the efficiency of alternative therapeutic molecules.

Breast cancer affects about one million times per year, and represents the most common form of cancer in females, affecting approximately 10% of all women at some stage of their life in the Western world. Men can also develop breast cancer, although their risk is less than 1 in 1000.

World Health Organization has planed that before 2010 the cancers will be the first causes of mortality worldwide, before the cardiovascular diseases. The last incidence and mortality data in France show the same evolution (Communication 2008 of Francim, the Institut de Veille Sanitaire, the Hospice Civils of Lyon and the Institut National du Cancer).

In breast cancers, it is essential to treat the patients depending on the biopathologic identity of their tumour. The goal is thus to provide reliable predictive factors to choose a targeted chimiotherapy, only to chimiosensitive patients and to choose specific drugs depending on specific biomarker of the tumour. Thus, the efficiency of the chimiotherapy depends on cellular characteristics and the presence of sensitivity biomarkers, like hormonal receptors, the expression of UPA, PAI1, Her2 and the topoisomerase II α. The tumour suppressor p53 is the most mutated gene in human tumours. Nevertheless, the mutations which affect the p53 gene should be carefully interpreted, particularly since its predictive value depends on breast cancer types.

The inventors have now discovered that the pattern of expression of a suppressor tumour p53 isoforms due to an aberrant epissage, are associated with a bad prognostic.

### Summary of the invention

The present invention relates to a method of testing a subject thought to be predisposed to having cancer which comprises the step of i) analyzing a biological sample from said subject for detecting the presence, the expression level or the absence of a p53 isoform selected in the group consisting in Δ133p53, Δ133p53γ and Δ133p53β, the presence of said p 53 isoform being indicative of cancer.

The presence or the absence of p53 isoforms can be evaluated by detecting a p53 isoform polypeptide or a fragment thereof, or by detecting a p53 isoform mRNA or a fragment thereof.

Furthermore, said method can include a further step of comparing the detected expression level of the p53 isoform with a positive or a negative control. For example, an expression level higher than a negative control is indicative of cancer.

Preferably, the methods according to the invention are *in vitro* methods.

Preferably, said cancer is a metastatic cancer.

In another aspect, the invention relates to a method for measuring the aggressiveness of cancer in a subject, which comprises the step of i) determining the presence, the level of expression or the absence of a p53 isoform selected in the group consisting in Δ133p53, Δ133p53γ and Δ133p53β in a biological sample from the subject, the presence of said isoform being indicative of an aggressive cancer, preferably said isoform being indicative of a metastatic cancer.

In a further aspect, the present invention relates to a method for determining the subject's response to an anti-cancer therapy, said subject is receiving or has received therapy for a state associated with cancer, which comprises the step of:
i) determining the expression level of a p53 isoform selected in the group consisting in Δ133p53, Δ133p53γ and Δ133p53β in a biological sample from the subj ect and
ii) comparing it to a control.

Preferably, this method includes a further step of analysing if the subject responds to therapy.

For example, the presence of said isoform is indicative of the subject's response to the therapy when the expression level of said p53 isoform is lower than the expression level measured in a sample from the subject before said subject has received therapy. Alternatively, the presence of said p53 isoform is indicative of the subject's response to the therapy when the expression level of said p53 isoform is equal or lower than the expression level measured in a sample from the subject which does not have a cancer.

The above method of the invention can be used in combination with other methods of cancer diagnosis or prognosis.

As used herein, the term "p53 isoforms" refers to a polypeptide or a mRNA which differs from the p53 polypeptide of sequence SEQ ID:7 or from the p53 mRNA having a cDNA sequence SEQ ID NO: 8 respectively, due to a splicing default. The p53 isoforms of the present invention is selected in the group consisting in Δ133p53, Δ133p53γ and Δ133p53β.

Preferably, said p53 isoform is Δ133p53β.

Preferably, Δ133p53β polypeptide is represented by the sequence SEQ ID:1, and the Δ133p53β mRNA has a cDNA sequence SEQ ID NO:2.

Preferably, Δ133p53 polypeptide is represented by the sequence SEQ ID:3, and the Δ133p53 mRNA has a cDNA sequence SEQ ID NO:4.

Preferably, Δ133p53γ polypeptide is represented by the sequence SEQ ID:5, and the Δ133p53γ mRNA has a cDNA sequence SEQ ID NO:6.

"Cancer" includes a malignant neoplasm characterized by deregulated or uncontrolled cell growth. The term "cancer" includes primary malignant tumours (e. g., those whose cells have not migrated to sites in the subject's body other than the site of the original tumor) and secondary malignant tumors (e. g., those arising from metastasis, the migration of tumour cells to secondary sites that are different from the site of the original tumour).

In the methods of the present invention, such cancer is preferably selected from the group consisting of breast cancer, ovarian cancer, digestive cancers and throat cancer is preferred, particularly of human subject, the more preferred is breast cancer or a colon cancer, and even more preferably a breast cancer.

The term splicing process consists of eliminating introns in pre-messenger RNAs to produce mature messenger RNAs that can be used by the translation mechanism of the cell (SHARP, Cell, vol. 77, p. 805-815, 1994). In the case of alternative splicing, the same precursor can be the source of messenger RNAs coding for proteins with distinct functions (BLACK, Annu. Rev. Biochem. vol. 72, p. 291-336, 2003).

"Splicing default" means an abnormal splicing process which can lead to the formation of abnormal isoforms.

The term "aggressive" (or "invasive") as used herein with respect to cancer refers to the proclivity of a tumour for expanding beyond its boundaries into adjacent tissue, or to the characteristic of the tumour with respect to metastasis. Invasive cancer can be contrasted with organ-confined cancer. For example, a basal cell carcinoma of the skin is a non-invasive or minimally invasive tumour, confined to the site of the primary tumour and expanding in size, but not metastasizing. In contrast, the cancer melanoma is highly invasive of adjacent and distal tissues. The invasive property of a tumour is often accompanied by the elaboration of proteolytic enzymes, such as collagenases, that degrade matrix material and basement membrane material to enable the tumor to expand beyond the confines of the capsule, and beyond confines of the particular tissue in which that tumor is located.

The term "metastasis" or "metastatic" as used herein refers to the condition of spread of cancer from the organ of origin to additional distal sites in the patient. The process of tumor metastasis is a multistage event involving local invasion and destruction of intercellular matrix, intravasation into blood vessels, lymphatics or other channels of transport, survival in the circulation, extravasation out of the vessels in the secondary site and growth in the new location.

The term "subject" includes mammals, e. g., humans, dogs, cows, horses, kangaroos, pigs, sheep, goats, cats, mice, rabbits, rats, and transgenic non-human animals, preferably human subject, and more preferably a woman.

The term "biological samples" includes solid and body fluid samples. The biological samples of the present invention may include cells, protein, blood or biological fluids such as bone marrow, ascites fluid or brain fluid (e. g., cerebrospinal fluid). Examples of solid biological samples include samples taken from feces, the rectum, central nervous system, bone, breast tissue, renal tissue, the uterine cervix, the endometrium, the head/neck, the gallbladder, parotid tissue, the prostate, the brain, the pituitary gland, kidney tissue, muscle, the oesophagus, the stomach, the small intestine, the colon, the liver, the spleen, the pancreas, thyroid tissue, heart tissue, lung tissue, the bladder, adipose tissue, lymph node tissue, the uterus, ovarian tissue, adrenal tissue, testis tissue, the tonsils, and the thymus. Examples of "body fluid samples" include samples taken from the blood, serum, semen, prostate fluid, seminal fluid, urine, saliva, sputum, mucus, bone marrow, lymph, and tears. Samples for use in the assays of the invention can be obtained by standard methods including venous puncture and surgical biopsy. In one embodiment, the biological sample is a breast tissue sample obtained by needle biopsy.

In a preferred embodiment, the biological sample used in the methods of the present invention is selected from the group consisting of bone marrow, serum, plasma, blood, lymph, or cells from the cancerous or suspected cancerous tissue or adjacent tissue thereof, preferably a bone marrow sample.

The term "expression level" or "level" means the concentration of a product of expression, e.g. a polypeptide or mRNA, in a sample.

As described in more detail below, the detection methods of the invention can be used to detect the mRNA of a p53 isoform of the present invention, the polypeptide of a p53 isoform of the present invention or specific fragments thereof, in a biological sample *in vitro.* For example, *in vitro* techniques for detection of said p53 isoform mRNA include Northern hybridizations and in situ hybridizations. *In vitro* techniques for detection of Δ133p53β polypeptide include immunohistochemistry, enzyme linked immunosorbent assays (ELISAs), Western blots, immunoprecipitations, and immunofluorescence.

In a preferred embodiment, the invention relates to the methods according to the present invention, wherein the p53 isoform is Δ133p53β.

When the methods according to the present invention are based on the detection or the quantification of a p53 isoform mRNA, it is also preferred that the determination of the presence, or the expression level, or the absence of said mRNA comprises a further step of:
amplifying said p53 isoform mRNA or cDNA, the complementary sequence thereof, or a fragment thereof having at least 10 nucleotides length which is specific of said p53 isoform.

As used herein, the term "mRNA" refers to a mature mRNA, i.e. which has already undergone the splicing event.

As used herein, the term "cDNA" shall refer to the DNA copy of the mRNA.

It is also preferred that the step of amplifying Δ133p53β mRNA or cDNA is performed by PCR or RT-PCR reaction.

This detection may be accomplished by isolating mRNA from a sample. When using mRNA detection, the method may be carried out by converting the isolated mRNA to cDNA according to standard methods; treating the converted cDNA with amplification reaction reagents (such as cDNA PCR reaction reagents) in a container along with an appropriate mixture of nucleic acid primers; reacting the contents of the container to produce amplification products; and analyzing the amplification products to detect the presence of a specific nucleic acid of a p53 isoform of the present invention in the biological sample.

Preferably, said primers have the sequence SEQ ID NO:9 and SEQ ID NO:10.

The term "primer", as used herein, refers to an oligonucleotide, whether occurring naturally (as in a purified restriction digest) or produced synthetically, and which is capable of initiating synthesis of a strand complementary to a nucleic acid when placed under appropriate conditions, i.e., in the presence of nucleotides and an inducing agent, such as a DNA polymerase, and at a suitable temperature and pH. The primer may be either single-stranded or doublestranded and must be sufficiently long to prime the synthesis of the desired extension product in the presence of the inducing agent. The exact length of the primer will depend upon many factors, including temperature, sequence and/or homology of primer and the method used. For example, in diagnostic applications, the oligonucleotide primer typically contains 10 to 25 or more nucleotides, depending upon the complexity of the target sequence, although it may contain fewer nucleotides.

The primers herein are selected to be "substantially" complementary to particular target DNA sequences. This means that the primers must be sufficiently complementary to hybridize with their respective strands. Therefore, the primer sequence need not reflect the exact sequence of the template. For example, a non-complementary nucleotide fragment (i.e., containing a restriction site) may be attached to the 5' end of the primer, with the remainder of the primer sequence being complementary to the strand. Alternatively, non-complementary bases or longer sequences can be interspersed into the primer, provided that the primer sequence has sufficient complementary with the sequence to hybridize therewith and form the template for synthesis of the extension product.

"Amplifying" refers to template-dependent processes and vector-mediated propagation which result in an increase in the concentration of a specific nucleic acid molecule relative to its initial concentration, or in an increase in the concentration of a detectable signal. As used herein, the term template-dependent process is intended to refer to a process that involves the template-dependent extension of a primer molecule.

The term template dependent process refers to nucleic acid synthesis of an RNA or a DNA molecule wherein the sequence of the newly synthesized strand of nucleic acid is dictated by the well-known rules of complementary base pairing (see, for example, Watson, J. D. et al., In: Molecular Biology of the Gene, 4th Ed., W. A. Benjamin, Inc., Menlo Park, Calif. (1987). Typically, vector mediated methodologies involve the introduction of the nucleic acid fragment into a DNA or RNA vector, the clonal amplification of the vector, and the recovery of the amplified nucleic acid fragment. Examples of such methodologies are provided by Maniatis T. et al., Molecular Cloning (A Laboratory Manual), Cold Spring Harbor Laboratory, 1982.

A number of template dependent processes are available to amplify the target sequences of interest present in a sample. One of the best known amplification methods is the polymerase chain reaction (PCR) which is described in detail in Mullis et al., US Patent No. 4,683,195, Mullis et al., US Patent No. 4,683,202, and Mullis et al., US Patent No. 4,800,159, and in Innis et al., PCR Protocols, Academic Press, Inc., San Diego Calif., 1990. Briefly, in PCR, two primer sequences are prepared which are complementary to regions on opposite complementary strands of the target sequence. An excess of deoxynucleoside triphosphates are added to a reaction mixture along with a DNA polymerase (e. g., Taq polymerase). If the target sequence is present in a sample, the primers will bind to the target and the polymerase will cause the primers to be extended along the target sequence by adding on nucleotides. By raising and lowering the temperature of the reaction mixture, the extended primers will dissociate from the target to form reaction products, excess primers will bind to the target and to the reaction products and the process is repeated. Preferably a reverse transcriptase PCR amplification procedure may be performed in order to quantify the amount of mRNA amplified. Polymerase chain reaction methodologies are well known in the art.

Still other amplification methods described in GB Application No. 2 202 328, and in PCT Application No. PCT/US 89/01025 may be used in accordance with the present invention. In the former application, "modified" primers are used in a PCR like, template and enzyme dependent synthesis. The primers may be modified by labelling with a capture moiety (e. g., biotin) and/or a detector moiety (e. g., enzyme). In the latter application, an excess of labelled probes are added to a sample. In the presence of the target sequence, the probe binds and is cleaved catalytically. After cleavage, the target sequence is released intact to be bound by excess probe. Cleavage of the labelled probe signals the presence of the target sequence.

Following amplification, the presence or absence of the amplification product may be detected. The amplified product may be sequenced by any method known in the art.

In another embodiment, the invention relates to the methods according to the present invention, wherein the determination of the presence or the absence of a p53 isoform selected in the group consisting in Δ133p53, Δ133p53γ and Δ133p53γ is carried out by a probe capable of specifically hybridizing with said p53 isoform mRNA, complementary sequence thereof or a fragment thereof having at least 10, 15, 20, 25, 30, 35, 40, 45, 50 or more nucleotides length which is specific of said p53 isoform, preferably hybridizing with human Δ133p53β mRNA, and even more preferably with Δ133p53β mRNA having the sequence SEQ ID NO: 2,.

Advantageously, the probe is chosen in the group consisting in sequences SEQ ID NO:9, SEQ ID NO:10 or SEQ ID NO:11, preferably sequence SEQ ID NO:11.

In a particular embodiment, the invention relates to the methods according to the present invention, wherein the determination of the presence, the expression level or the absence of Δ133p53β mRNA is carried out by the following method comprising the steps of:

(i) contacting a nucleic acid probe specific of a p53 isoform selected in the group consisting in Δ133p53, Δ133p53γ and Δ133p53β, under hybridizing conditions with the biological test sample comprising either;

(ii) RNA molecules isolated from a biological sample of the human subject, wherein the biological sample is suspected of containing tumour cells, or

(ii) nucleic acid molecules synthesized from the isolated RNA molecules as cDNA;

wherein the nucleic acid probe has a nucleotide sequence comprising either a fragment of at least 15 nucleotides length of the sequence of the p53 isoform, or a fragment thereof, or their complement, and

(b) detecting the formation of hybrids of the nucleic acid probe and the test sample;

wherein the presence of hybrids indicates the presence of tumour cells in the tissue obtained from the human subject.

Probes based on the sequence of a nucleic acid molecule of the invention can be used to detect transcripts corresponding to mRNA of p53 isoform selected in the group consisting in Δ133p53, Δ133p53γ and Δ133p53β. The nucleic acid probe can be, for example, a full-length cDNA, or a fragment thereof, such as an oligonucleotide having a length sufficient to specifically hybridize under stringent conditions to mRNA of p53 isoform of the invention. Hybridization of a mRNA with the probe indicates that the marker in question is being expressed. In an embodiment, the probe includes a label group attached thereto, e. g., a radioisotope, a fluorescent compound, an enzyme, or an enzyme co-factor.

In one format, the mRNA is immobilized on a solid surface and contacted with a probe, for example by running the isolated mRNA on an agarose gel and transferring the mRNA from the gel to a membrane, such as nitrocellulose. In an alternative format, the probe(s) are immobilized on a solid surface and the mRNA is contacted with the probe (s), for example, in an Affymetrix gene chip array. A skilled artisan can readily adapt known mRNA detection methods for use in detecting the expression level of mRNA encoded by the markers of the present invention.

When the methods according to the present invention are based on the detection or the quantification of a p53 isoform polypeptide expression level, it is also preferred that the determination of the presence, or the expression level, or the absence of said p53 isoform by an immunohistochemical or an immunoassay method using an antibody capable of specifically recognizing said p53 isoform.

In one embodiment, the determination of the presence, the level expression or the absence of a p53 isoform selected in the group consisting in Δ133p53, Δ133p53γ and Δ133p53β, is carried out by contacting the biological sample with an antibody specific of a p53 isoform selected in the group consisting in Δ133p53, Δ133p53γ and Δ133p53β polypeptide or a fragment thereof; determining the binding of the antibody to the biological sample.

"Antibody" includes immunoglobulin molecules and immunologically active determinants of immunoglobulin molecules, i.e., molecules that contain an antigen binding site (epitope) which specifically binds (immunoreacts with) an antigen. Specificity of binding in the large and diverse set of antibodies is found in the variable (V) determinant of the H and L chains. Antibody includes polyclonal antibodies, monoclonal antibodies, whole immunoglobulins, and antigen binding fragments of the immunoglobulins.

The binding sites of the proteins that comprise an antibody, i.e., the antigen-binding functions of the antibody, are localized by analysis of fragments of a naturally occurring antibody. Thus, antigen-binding fragments are also intended to be designated by the term "antibody" Examples of binding fragments encompassed within the term antibody include: a Fab fragment consisting of the VL, VH, CL and Cm domains; an Fd fragment consisting of the VH and CHI domains; an Fv fragment consisting of the VL and VH domains of a single arm of an antibody; an isolated complementarity determining region (CDR); and an F(ab') 2 fragment, a bivalent fragment comprising two Fab' fragments linked by a disulfide bridge at the hinge region. These antibody fragments are obtained using conventional techniques well-known to those with skill in the art, and the fragments are screened for utility in the same manner as are intact antibodies. The term "antibody" is further intended to include bispecific and chimeric molecules having at least one antigen binding determinant derived from an antibody molecule.

In the diagnostic and prognostic assays of the invention, the antibody can be a polyclonal antibody or a monoclonal antibody, and monoclonal antibodies are preferred.

Preferrably, said antibody is labelled.

Polyclonal antibodies are produced by immunizing animals, usually a mammal, by multiple subcutaneous or intraperitoneal injections of an immunogen (antigen) and an adjuvant as appropriate. As an illustrative embodiment, animals are typically immunized against a protein, peptide or derivative by combining about 1 µg to 1 mg of protein capable of eliciting an immune response, along with an enhancing carrier preparation, such as Freund's complete adjuvant, or an aggregating agent such as alum, and injecting the composition intradermally at multiple sites. Animals are later boosted with at least one subsequent administration of a lower amount, as 1/5 to 1/10 the original amount of immunogen in Freund's incomplete adjuvant (or other suitable adjuvant) by subcutaneous injection at multiple sites. Animals are subsequently bled, serum assayed to determine the specific antibody titer, and the animals are again boosted and assayed until the titer of antibody no longer increases (i.e., plateaus).

Such populations of antibody molecules are referred to as "polyclonal" because the population comprises a large set of antibodies each of which is specific for one of the many differing epitopes found in the immunogen, and each of which is characterized by a specific affinity for that epitope. An epitope is the smallest determinant of antigenicity, which for a protein may comprise a peptide of six to eight residues in length (Berzofsky J. and 1. Berkower (1993) in Paul, W., Ed., Fundamental Immunology, Raven Press, N. Y., p. 246). Affinities range from low, e. g. 10⁻⁶ M, to high, e. g., 10⁻¹¹.

The polyclonal antibody fraction collected from mammalian serum is isolated by well known techniques, e. g. by chromatography with an affinity matrix that selectively binds immunoglobulin molecules such as protein A, to obtain the IgG fraction. To enhance the purity and specificity of the antibody, the specific antibodies may be further purified by immunoaffinity chromatography using solid phase-affixed immunogen. The antibody is contacted with the solid phase-affixed immunogen for a period of time sufficient for the immunogen to immunoreact with the antibody molecules to form a solid phase-affixed immunocomplex. Bound antibodies are eluted from the solid phase by standard techniques, such as by the use of buffers of decreasing pH or increasing ionic strength, the eluted fractions are assayed, and those containing the specific antibodies are combined.

"Monoclonal antibody" as used herein refers to a preparation of antibody molecules of single molecular composition. A monoclonal antibody composition displays a single binding specificity and affinity for a particular epitope. Monoclonal antibodies can be prepared using a technique which provides for the production of antibody molecules by continuous growth of cells in culture. These include but are not limited to the hybridoma technique originally described by Kohler and Milstein (1975, Nature, 256: 495-497; see also Brown et al., 1981, J. Immunol., 127:539-46; Brown et al., 1980, J. Biol. Chem., 255:4980-83; Yeh et al., 1976, PNAS 76:2927-31; and Yeh et al., 1982, Int. J. Cancer, 29:269-75) and the more recent human B cell hybridoma technique (Kozbor et al., 1983, Immunol. Today 4:72), EBV-hybridoma technique (Cole et al., 1985, Monoclonal Antibodies and Cancer Therapy, Alan R. Liss, Inc., pp. 77-96), and trioma techniques. The technology for producing hybridomas is well known (see generally Current Protocols in Immunology, Coligan et al. ed., John Wiley & Sons, New York, 1994). Hybridoma cells producing a monoclonal antibody of the invention are detected by screening the hybridoma culture supernatants for antibodies that bind the polypeptide of interest, e. g., using a standard ELISA assay.

A monoclonal antibody can be produced by the following steps. In all procedures, an animal is immunized with an antigen such as a protein (or peptide thereof) as described above for preparation of a polyclonal antibody. The immunization is typically accomplished by administering the immunogen to an immunologically competent mammal in an immunologically effective amount, i.e., an amount sufficient to produce an immune response. Preferably, the mammal is a rodent such as a rabbit, rat or mouse. The mammal is then maintained on a booster schedule for a time period sufficient for the mammal to generate high affinity antibody molecules as described. After a sufficient time to generate high affinity antibodies, the animal (e. g., mouse) is sacrificed and antibody-producing lymphocytes are obtained from one or more of the lymph nodes, spleens and peripheral blood. Spleen cells are preferred, and can be mechanically separated into individual cells in a physiological medium using methods well known to one of skill in the art. The antibody-producing cells are immortalized by fusion to cells of a mouse myeloma line.

Mouse lymphocytes give a high percentage of stable fusions with mouse homologous myelomas, however rat, rabbit and frog somatic cells can also be used. Spleen cells of the desired antibody-producing animals are immortalized by fusing with myeloma cells, generally in the presence of a fusing agent such as polyethylene glycol. Any of a number of myeloma cell lines suitable as a fusion partner can be used with to standard techniques, for example, the P3-NS1/1-Ag4-1, P3-x63-Ag8.653 or Sp2/O-Ag14 myeloma lines, available from the American Type Culture Collection (ATCC), Rockville, MD.

The fusion-product cells, which include the desired hybridomas, are cultured in selective medium such as HAT medium, designed to eliminate unfused parental myeloma or lymphocyte or spleen cells. Hybridoma cells are selected and are grown under limiting dilution conditions to obtain isolated clones. The supernatants of each clonal hybridoma is screened for production of antibody of desired specificity and affinity, e. g., by immunoassay techniques to determine the desired antigen such as that used for immunization. Monoclonal antibody is isolated from cultures of producing cells by conventional methods, such as ammonium sulfate precipitation, ion exchange chromatography, and affinity chromatography (Zola et al., Monoclonal Hybridoma Antibodies: Techniques And Applications, Hurell (ed.), pp. 51-52, CRC Press, 1982).

Hybridomas produced according to these methods can be propagated in culture *in vitro* or *in vivo* (in ascites fluid) using techniques well known to those with skill in the art.

Alternative to preparing monoclonal antibody-secreting hybridomas, a monoclonal antibody directed against a polypeptide of the invention can be identified and isolated by screening a recombinant combinatorial immunoglobulin library (e. g., an antibody phage display library) with the polypeptide of interest. Kits for generating and screening phage display libraries are commercially available (e. g., the Pharmacia Recombinant Phage Antibody System, Catalog No. 27-9400-01; and the Stratagene SutfZ4P Phage Display Kit, Catalog No. 240612). Additionally, examples of methods and reagents particularly amenable for use in generating and screening an antibody display library can be found in, for example, US Patent No. 5,223,409; PCT Publication No. WO 92/18619; PCT Publication No. WO 91/17271; PCT Publication No. WO 92/20791; PCT Publication No. WO 92/15679; PCT Publication No. WO 93/01288; PCT Publication No. WO 92/01047; PCT Publication No. WO 92/09690; PCT Publication No. WO 90/02809; Fuchs et al. (1991) Bio/Technology 9:1370-1372; Hay et al. (1992) Hum. Antibod. Hybridomas, 3:81-85; Huse et al. (1989) Science 246:1275-1281 ; Griffiths et al. (1993) EMBO J., 12: 725-734.

Additionally, recombinant antibodies, such as chimeric and humanized monoclonal antibodies, comprising both human and non-human portions, which can be made using standard recombinant DNA techniques, are within the scope of the invention. Such chimeric and humanized monoclonal antibodies can be produced by recombinant DNA techniques known in the art, for example using methods described in PCT Publication No. WO 87/02671; European Patent Application 0 184 187; European Patent Application 0 171 496; European Patent Application 0 173 494; PCT Publication No. WO 86/01533; U. S. Patent No. 4,816,567; European Patent Application 0 125 023; Better et al. (1988) "Labelled antibody" as used herein includes antibodies that are labeled by a detectable means and includes enzymatically, radioactively, fluorescently, chemiluminescently, and/or bioluminescently labeled antibodies by any of the many different methods known to those skilled in this art.

One of the ways in which an antibody can be detectably labelled is by linking the same to an enzyme. This enzyme, in turn, when later exposed to its substrate, will react with the substrate in such a manner as to produce a chemical moiety which can be detected, for example, by spectrophotometric, fluorometric or by visual means. Enzymes which can be used to detectably label a p53 isoform-specific antibody include, but are not limited to, malate dehydrogenase, staphylococcal nuclease, delta-V-steroid isomerase, yeast alcohol dehydrogenase, alpha-glycerophosphate dehydrogenase, triose phosphate isomerase, horseradish peroxidase, alkaline phosphatase, asparaginase, glucose oxidase, beta-galactosidase, ribonuclease, urease, catalase, glucose-VI-phosphate dehydrogenase, glucoamylase and acetylcholinesterase.

Detection may be accomplished using any of a variety of immunoassays. For example, by radioactively labelling an antibody, it is possible to detect the antibody through the use of radioimmune assays. A description of a radioimmune assay (RIA) may be found in Laboratory Techniques and Biochemistry in Molecular Biology, by Work T. S. et al., North Holland Publishing Company, NY (1978), with particular reference to the chapter entitled "An Introduction to Radioimmune Assay and Related Techniques" by Chard T. The radioactive isotope can be detected by such means as the use of a gamma counter or a scintillation counter or by audioradiography. Isotopes which are particularly useful for the purpose of the present invention are: ³H, ¹³¹I, ³⁵S, 14C, and preferably ¹²⁵I.

It is also possible to label an antibody with a fluorescent compound. When the fluorescently labelled antibody is exposed to light of the proper wave length, its presence can then be detected due to fluorescence. Among the most commonly used fluorescent labelling compounds are fluorescein isothiocyanate, rhodamine, phycoerytherin, phycocyanin, allophycocyanin, ophthaldehyde and fluorescamine.

An antibody can also be detectably labelled using fluorescence emitting metals such as ¹⁵²Eu, or others of the lanthanide series. These metals can be attached to the antibody using such metal chelating groups as diethylenetriaminepentaacetic acid (DTPA) or ethylenediaminetetraacetic acid (EDTA).

An antibody can also be detectably labelled by coupling it to a chemiluminescent compound. The presence of the chemiluminescent-tagged antibody is then determined by detecting the presence of luminescence that arises during the course of a chemical reaction. Examples of particularly useful chemiluminescent labelling compounds are luminol, luciferin, isoluminol, theromatic acridinium ester, imidazole, acridinium salt and oxalate ester.

Likewise, a bioluminescent compound may be used to label an antibody of the present invention. Bioluminescence is a type of chemiluminescence found in biological systems in which a catalytic protein increases the efficiency of the chemiluminescent reaction. The presence of a bioluminescent protein is determined by detecting the presence of luminescence. Important bioluminescent compounds for purposes of labelling are luciferin, luciferase and aequorin.

In the detection assays of the invention, the amount of binding of the antibody to the biological sample can be determined by the intensity of the signal emitted by the labelled antibody and/or by the number cells in the biological sample bound to the labelled antibody.

The detection or the expression level of a p53 isoform selected in the group consisting in Δ133p53, Δ133p53γ and Δ133p53β in a biological sample may be determined by a radioimmunoassay, an immunoradiometric assay, and/or an enzyme immunoassay.

"Radioimmunoassay" is a technique for detecting and measuring the concentration of an antigen using a labelled (i.e. radioactively labelled) form of the antigen (i.e. Δ133p53β polypeptide). Examples of radioactive labels for antigens include ³H, ¹⁴C, and ¹²⁵I. The concentration of the p53 isoform in a biological sample is measured by having the antigen in the sample compete with a labelled (i.e. radioactively) antigen for binding to an antibody to the antigen. To ensure competitive binding between the labelled antigen and the unlabeled antigen, the labelled antigen is present in a sufficient concentration to saturate the binding sites of the antibody. The higher the concentration of antigen in the sample, the lower the concentration of labelled antigen that will bind to the antibody will be.

In a radioimmunoassay, to determine the concentration of labelled antigen bound to an antibody, the antigen-antibody complex must be separated from the free antigen. One method for separating the antigen-antibody complex from the free antigen is by precipitating the antigen-antibody complex with an anti-isotype antiserum. Another method for separating the antigen-antibody complex from the free antigen is by precipitating the antigen-antibody complex with formalin-killed *S*. *aureus.* Yet another method for separating the antigen-antibody complex from the free antigen is by performing a "solid-phase radioimmunoassay" where the antibody is linked (i.e. covalently) to Sepharose beads, polystyrene wells, polyvinylchloride wells, or microtiter wells. By comparing the concentration of labelled antigen bound to antibody to a standard curve based on samples having a known concentration of antigen, the concentration of antigen in the biological sample can be determined.

An "Immunoradiometric assay" (IRMA) is an immunoassay in which the antibody reagent is radioactively labeled. An IRMA requires the production of a multivalent antigen conjugate by techniques such as conjugation to a protein e.g., rabbit serum albumin (RSA). The multivalent antigen conjugate must have at least 2 antigen residues per molecule and the antigen residues must be of sufficient distance apart to allow binding by at least two antibodies to the antigen. For example, in an IRMA the multivalent antigen conjugate can be attached to a solid surface such as a plastic sphere.

Unlabeled "sample" antigen and antibody to antigen which is radioactively labelled are added to a test tube containing the multivalent antigen conjugate coated sphere. The antigen in the sample competes with the multivalent antigen conjugate for antigen antibody binding sites. After an appropriate incubation period, the unbound reactants are removed by washing and the amount of radioactivity on the solid phase is determined. The amount of bound radioactive antibody is inversely proportional to the concentration of antigen in the sample.

The most common enzyme immunoassay is the "Enzyme-Linked Immunosorbent Assay (ELISA)". The "Enzyme-Linked Immunosorbent Assay (ELISA)" is a technique for detecting and measuring the concentration of an antigen using a labelled (i.e. enzyme linked) form of the antibody.

In a "sandwich ELISA", an antibody (i.e. anti- Δ133p53β peptide) is linked to a solid phase (i.e. a microtiter plate) and exposed to a biological sample containing antigen (i.e. Δ133p53β peptide). The solid phase is then washed to remove unbound antigen. A labelled (i.e. enzyme linked) is then bound to the bound-antigen (if present) forming an antibody-antigen-antibody sandwich. Examples of enzymes that can be linked to the antibody are alkaline phosphatase, horseradish peroxidase, luciferase, urease, and 3-galactosidase. The enzyme linked antibody reacts with a substrate to generate a colored reaction product that can be assayed for.

In a "competitive ELISA", antibody is incubated with a sample containing antigen (i.e. a p53 isoform peptide). The antigen-antibody mixture is then contacted with an antigen-coated solid phase (i.e. a microtiter plate). The more antigen present in the sample, the less free antibody that will be available to bind to the solid phase. A labelled (i.e. enzyme linked) secondary antibody is then added to the solid phase to determine the amount of primary antibody bound to the solid phase.

In an "immunohistochemistry assay" a section of tissue is tested for specific proteins by exposing the tissue to antibodies that are specific for the protein that is being assayed. The antibodies are then visualized by any of a number of methods to determine the presence and amount of the protein present. Examples of methods used to visualize antibodies are, for example, through enzymes linked to the antibodies (e. g., luciferase, alkaline phosphatase, horseradish peroxidase, or P-galactosidase), or chemical methods (e.g., DAB/Substrate chromagen) or gold, fluorescent or labelled antibodies by any of the many different methods known to those skilled in this art.

In another aspect, the present invention concerns a method of screening potential anti-cancer compounds, which comprises the steps of:
a) optionally measuring in a cell known to express a p53 isoform selected in the group consisting in Δ133p53, Δ133p53γ and Δ133p53β, the expression level of said p53 isoform;
b) contacting the compound to be tested with said cell;
c) determining the expression level of said p53 isoform by a method of detecting said p53 isoform as described above; and

selecting said compound as a potential anti-cancer compound if the p53 isoform is not expressed in the cell, or has an expression level lower than before step a).

Preferably, the compounds to be tested are antisense RNA or interfering RNA (iRNA).

In another aspect, the present invention is directed to a method for the production of polyclonal antibodies specifically recognizing a p53 isoform polypeptide selected in the group consisting in Δ133p53, Δ133p53γ and Δ133p53β polypeptide, wherein such method comprises the step of:
a) immunization of a mammal animal with a immunologically effective amount of a p53 isoform polypeptide selected in the group consisting in Δ133p53, Δ133p53γ and Δ133p53β, or against a specific epitope fragment of at least 9 amino acids length of said polypeptide, optionally with an enhancing carrier preparation;
b) optionally, *in vitro* determining the presence of specific antibodies in the animal serum or plasma; and
c) purifying or isolating the specific anti- Δ133p53, anti- Δ133p53γ or anti-Δ133p53β polypeptide from the animal serum or plasma.

It also forms part of the present invention a method for the production of an hybridoma cell capable of secreting monoclonal antibodies specifically recognizing a p53 isoform selected in the group consisting in Δ133p53, Δ133p53γ and Δ133p53β polypeptides, wherein such method comprises the step of:
a) immunization of a mammal animal with a immunologically effective amount of a p53 isoform selected in the group consisting in Δ133p53, Δ133p53γ and Δ133p53β, or against an epitope fragment of at least 9 amino acids length of said polypeptide, optionally with an enhancing carrier preparation;

b) isolating antibodies anti- p53 isoform peptide producing lymphocytes from the spleen, lymph nodes or peripheral blood of that mammal animal; and

c) immortalizing the antibodies anti-Δ133p53, anti- Δ133p53γ or anti-Δ133p53β polypeptide producing lymphocytes by fusion of said lymphocytes to cells of same species mammal animal myeloma line.

It also forms part of the present invention a method for the production of monoclonal antibodies specifically recognizing a p53 isoform selected in the group consisting in Δ133p53, Δ133p53γ and Δ133p53β polypeptide, wherein such method comprises the step of:
a) producing an hybridoma cell capable of secreting monoclonal antibodies specifically recognizing a p53 isoform selected in the group consisting in Δ133p53, Δ133p53γ and Δ133p53β polypeptides, according to the above method of the invention;
b) culturing such hybridoma cell in appropriate culture medium and culture conditions;
c) purifying or isolating from such culture medium the monoclonal antibodies which are secreted.

In another part, the present invention comprises polyclonal or monoclonal antibodies obtainable by the method for the production of polyclonal or monoclonal antibodies according to the present invention wherein such antibodies are specifically recognizing a p53 isoform selected in the group consisting in Δ133p53, Δ133p53γ and Δ133p53β.

In another aspect, the present invention relates to kit for the detection or quantification of a p53 isoform selected in the group consisting in Δ133p53, Δ133p53γ and Δ133p53β mRNA or polypeptide, wherein such kit comprises:
a) a polyclonal or monoclonal antibody according to the invention; or
b) a pair of primers selecting from the group consisting of a pair of primers capable of amplifying the sequence SEQ ID NO: 2, SEQ ID NO: 4 or SEQ ID NO: 6, or a fragment thereof having at least 10 nucleotides length; or
c) a probe having a nucleotide sequence comprising either a fragment of at least 10 nucleotides length of a sequence of a p53 isoform selected in the group consisting in Δ133p53, Δ133p53γ and Δ133p53β, or their complement.

For antibody-based kits, the kit can comprise, for example: (1) a first antibody (e. g., in solution or attached a solid support) which binds to a p53 isoform polypeptide according to the invention; and, optionally, (2) a second, different antibody conjugated to a detectable label which binds to either the p53 isoform polypeptide or to the first antibody.

For oligonucleotide-based kits, the kit can comprise, for example: (1) an oligonucleotide, e. g., a detectably labelled oligonucleotide, which hybridizes to p53 isoform nucleic acid sequence (mRNA or cDNA, or specific fragment thereof) or (2) a pair of primers useful for amplifying a nucleic acid molecule of a p53 isoform of the present invention. The kit can also comprise, e.g., a buffering agent, a preservative, or a protein stabilizing agent. The kit can further comprise components necessary for detecting the detectable label (e. g., an enzyme or a substrate). The kit can also contain a control sample or a series of control samples which can be assayed and compared to the biological sample. Each component of the kit can be enclosed within an individual container and all of the various containers can be within a single package, along with instructions for interpreting the results of the assays performed using the kit.

The present invention also concerns a method for the complement of morphological diagnosis by the detection or the quantification of a p53 isoform selected in the group consisting in Δ133p53, Δ133p53γ in biological sample of mammal, by the above-cited methods or by a method well known by the skilled man, such immunohistochemical or immunoassay for the detection of the above defined peptide or by any method detecting the above defined nucleotide sequence (e.g. RT-PCR, quantitative RT-PCR, FISH, etc.) in cancer of any type (e.g.: breast, colon, pancreas, head and neck, etc.).

### DESCRIPTION OF THE FIGURES

Figure 1 shows the disease free survival (figure 1a) and the survival fonctions (figure 1b) during the time in patients expressing Δ133p53β or not.
Figure 2 shows the disease free survival (figure 2a) and the survival fonctions (figure 2b) during the time in patients depending on the expression of Δ133p53β and ER.
Figure 3 shows the percentage of invasiveness (figure 3a) and the percentage of migration (figure 3b) depending on the Δ133p53β expression. Figure 3c is a schematic representation of p53 and Δ133p53β isoform (NLS: Nuclear Localisation Signal).
Figure 4a shows the quantative analysis of blebbing cells versus adherent cells in the GFP positive cells.

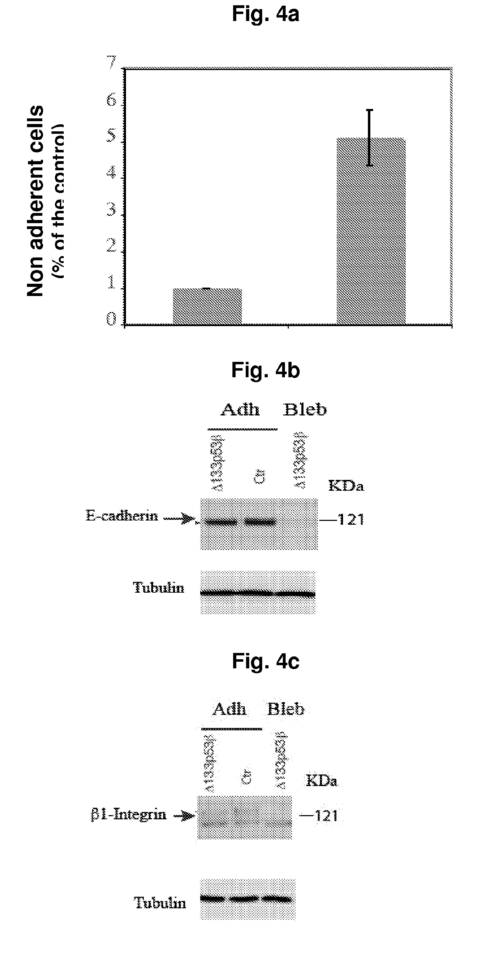

Figure 4 b and c shows t Western blot analysis of the expression of the myc-tagged constructs in cells used for E-cadherin and beta1-integrin western blotting. Adh: cells which are still adherent with the support; bleb: cells which show blebbing movement and which have detached from the support. Normalisation was performed using an anti-GAPDH antibody.

The following examples and the figures are given for the purpose of illustrating various embodiments of the invention and are not meant to limit the present invention in any fashion.

### EXAMPLES

### Example 1: Material and methods

### DNA constructs, reagents and antibodies :

Human p53 isoforms constructs were kindly provided by J.C. Bourdon. They were sub-cloned into the EcoRI and BamHI sites of pEGFPC1 (Clonetech) to give GFP-tagged proteins or in the BamHI and EcoRI sites of pLPCmyc to give myc-tagged proteins. Constructions were amplified using the Nucleobond PC 500 kit (Macherey-Nagel) according to the manufacturer's instructions.

Y27632 was purchased from calbiochem and was used at 10µM in all experiments.

The mouse anti-E-cadherin (clone 36), the mouse anti-beta1-integrin, the mouse anti-ROCK I and the mouse anti-ROCK II antibodies were purchased from BD-transduction laboratories and were diluted at 1/400°, 1/2500°, 1/250° and 1/250° respectively. The mouse anti-RhoA and the rabbit anti-ECT2 antibodies were purchased from Santa Cruz (26C4 and C-20 respectively) and were diluted at 1/500° and 1/200° respectively. The mouse anti-GEF-H1 antibody was kindly provided by K. Matter and was diluted at 1/50°. The rabbit anti-p53 antibodies (CM1) were kindly provided by J.C. Bourdon and were diluted at 1/1000°.

The Horse-Radish Peroxydase (HRP)-conjugated anti-IgG antibodies were purchased from GE-Healthcare and were diluted at 1/5000°.

The Western Lightning Chemiluminescence (ECL) reagents were purchased from PerkinElmer.

### Cell culture and transfections:

Hct116 cells were kindly provided by B. Vogelstein and were cultured at 37°C in the presence of 5% CO2 in McCOY'5A media (Sigma) supplemented with 10% foetal calf serum (FCS).

Transitory transfections of isoforms were done using JetPei kit (Qbiogen) according to the manufacturer's instructions: 9µg of DNA were used to transfect a 100 mm dish of 70% confluent cells. After 24 hours of transfection, cells were diluted at ½ and all experiments were done at 48 hours after transfection. Transitory transfections of siRNA at 50nM were done using Interferin (Polyplus) according to the manufacturer's instructions.

### Time-lapse imaging:

Time-lapse nomarski microscopy was performed on a Leica DMIRE2 inverted microscope with an automatic shutter and GFP filter sets, a 63x oil-immersion objective (HC x PL APO 1.32-0.6 oil CS), sample heater (37°C) and home-made CO2 incubation chamber. Images were captured with micromax CCD camera (1300Y/HS) imaging software, converted to TIFF files and were edited and compiled with metamorph. The exposure time was 500 ms for GFP and 300 ms for light. Images were captured every 3sec and during 5 minutes or every 4 min during 12 hours.

### FACS:

Cells transfected with the GFP-tagged isoforms of p53 which were not used for the invasion assays were spined at 1200 rpm for 5 minutes and fixed by adding 1mL of 70% EtOH at -20°C. Propidium iodide staining was performed and the number of GFP-expressing cells compared to total cells in each condition was quantified by FACS using the CellQuest software.

### Cell extracts, Western blotting:

Media containing blebbing cells was spined at 1200 rpm for 5 minutes and the pellet constituted of blebbing invasive cells was lysed. The remaining adherent cells were gently scrapped in lysis buffer. The two populations of cells were analyzed separately for each conditions (see figure legends). The amount of total proteins in each extract was quantified using BCA kit (promega). 8% SDS-PAGE gels were used to detect E-cadherin, betal-integrin, ROCK 1, ROCK 2, ECT2 and GEF-H1 and 12% SDS-PAGE gels were used to detect RhoA. Equal amount (30µg) of proteins was loaded on each lane. Proteins were then transfered electrophoretically on nitrocellulose (for p53, E-cadherin, beta1-integrin, ROCK 1, ROCK 2, ECT2 and GEF-H1) or PVDF (for GTP-RhoA) membrane. Membranes were blocked in TBS/0,1% Tween 20 containing 3% milk for one hour and then incubated overnight with the primary antibobies diluted in TBS/0,1% Tween 20 containing 3% milk. After several washes in TBS/Tween, membranes were incubated with anti-rabbit or anti-mouse Ig antibodies linked to HRP. Membranes were developped with ECL according to the manufacturer's instructions.

Scanned autoradiographs were quantified using AIDA/2D densitometry software.

### RhoA activity assay:

For RhoA activity assay, cells were lysed in 50 mM Tris, pH 7.2, 1% Triton X-100, 0.5% sodium deoxycholate, 500 mM NaCl, 10 mM MgCl2, 1 mM PMSF, and cocktail protease inhibitors. Cleared lysates were incubated with 25 µg of a commercial GST fusion protein containing the RhoA-binding domain of Rhotekin (GST-RBD, cytoskeleton) beads for 30 min at 4°C. Precipitated complexes were washed four times in Tris buffer containing 1% Triton X-100, 150 mM NaCl, 10 mM MgCl2, 0.1 mM PMSF, eluted in SDS sample buffer, immunoblotted and analyzed with antibodies specific for Rho A. An aliquot of the total lysate used for precipitation was run alongside to quantify total RhoA present in cell lysates. Scanned autoradiographs were quantified using Aida/2D densitometry software and normalized as a function of the expression of the RhoA protein.

### Invasion assays:

The quantification of cell invasion was carried out in Transwell cell culture chambers containing fluorescence-blocking polycarbonate porous membrane inserts (Fluoroblock ; #351152; BD Biosciences ; pore size 8 µm). 100 µl of 2mg/mL Matrigel with reduced growth factors (a commercially prepared reconstituted BM from Englebreth-Holm-Swarm tumours, # 354230; BD Biosciences) were prepared in a Transwell. Cells were transfected and treated or not with Y27632 as monolayers before trypsinization and plating (10.10⁴) in 2% FCS containing media on top of a thick layer (around 500 µm) of Matrigel contained within the upper chamber of a Transwell. Controls were left untreated. The upper and lower chambers were then filled with respectively 2% FCS containing media and media with 10 % FCS, thus establishing a soluble gradient of chemo-attractant that permits cell invasion throughout the Matrigel. Cells were allowed to invade at 37°C, 5% CO₂ through the gel before fixing for 15 min in 3.7 % formaldehyde. Cells that had invaded through the Matrigel were detected on the lower side of the filter by GFP fluorescence and counted for cell number. All the surface of the filter is counted and each assay was performed twice in triplicate for each condition.

### Breast cancer patients

Primary, previously untreated operable breast cancer from 171 Caucasian women (age range 24-89 years; median age 64 years) with sufficient tumour tissue surplus to diagnostic requirements and complete clinical and pathological data were analysed. Tumor tissue were macrodissected by a specialist breast pathologist and snap frozen in liquid nitrogen prior to storage at -80°C. Normal breast tissue was obtained from patients undergoing reduction mammoplasty who had no personal or family history of breast cancer. Samples were examined following Local Research Ethics Committee approval under delegated authority by the Tayside Tissue Bank.

### RT-PCR analysis

Approximately 10 mg of tumour tissue (>40% of tumour cells) was homogenized in 750µl QIAzol lysis reagent (Qiagen Ltd, Crawley, West Sussex, UK) and extracted RNA quality was confirmed using the BioAnalyzer 2100™ (Agilent Technologies, Palo Alto, CA, USA), demonstrating 28S/18S ≥1.5 with two sharp peaks confirmed prior to RT-PCR.

p53 isoform cDNA is too long to be specifically quantified by RT-qPCR, therefore we used a semi-quantitative RT-PCR method requiring high quality total RNA 28S/18S ratio > 1.5. After reverse-transcription of 500ng total RNA using random primers, actin cDNA was amplified by PCR to confirm reverse-transcription efficiency. 0.5µg of total RNA from each tumour sample was reverse-transcribed (AMV RT, 45C, random primer) and cDNA quality confirmed by amplification of actin by PCR in 30 cycles. p53 isoform cDNA was amplified by 2 nested PCR of 30 cycles using primers specific of each isoforms as previously described (Bourdon et al., 2005a). Tumours were considered to express each p53 isoform after sequencing of the corresponding PCR fragment.

### P53 mutation analysis

### AmpliChip p53 Test

The AmpliChip p53 Test is a product which is currently under development at Roche Molecular Systems, Inc (Pleasanton, California, USA). One 10-micron section of formalin-fixed paraffin-embedded tumor tissue or 100 ng purified genomic DNA from fresh frozen tumor is needed for this test. In this study, 100 ng genomic DNA extracted from homogenized frozen tissues were used for amplification of products encompassing the coding regions of the p53 gene in two reactions (A and B). Reaction A amplifies Exons 2, 5, 8, 10, exon 4 upstream sequences with an internal control while Reaction B amplifies for exons 3, 6, 7, 9, 11, exon 4 downstream sequences with the same internal control. The PCR products generated from the A and B reactions were combined for DNase I cleavage to generate small DNA fragments of an average size of 50-100 nucleotides. The fragmented DNA amplicons were subsequently labeled with biotin by Terminal Transferase. The biotin-labeled p53 target DNA fragments were added to the hybridization buffer. The mixture was hybridized to the oligonucleotides located on the AmpliChip p53 Microarray using the Affymetrix GeneChip Fluidics Station 450Dx and an AmpliChip p53 specific protocol. The hybridized AmpliChip p53 Microarray was washed and stained with a streptavidin-conjugated fluorescent dye (phycoerythrin). After staining, the AmpliChip p53 Microarray was scanned by an Affymetrix GeneChip Scanner 3000Dx using a laser that excites the fluorescent label bound to the hybridized p53 target DNA fragments. The amount of emitted light is proportional to bound target DNA at each location on the probe microarray

### Chip Design and Data Analysis of Microarray Signals

The AmpliChip p53 Microarray designed by Roche Molecular System consists of over 33,000 probe sets of more 220,000 individual oligonucleotides tiled for a total of 1268 nucleotide positions of coding regions of exons 2 - 11. A single probe set for an interrogating base position includes five probes, one probe to hybridize to the wild type, three probes to detect three possible single base pair mutations, and one probe to detect single deletion. There are at least 24 probe sets for each nucleotide position, including both sense and antisense probe sequences. AmpliChip p53 Microarrays are manufactured by Affymetrix using technology that combines photolithographic methods and combinatorial chemistry. The p53 mutation status was determined by a p53 Mutation Detection Algorithm developed by Roche Molecular System, which is designed to detect single base pair substitutions and single base pair deletions of a sample in a background of wild type p53 DNA probe intensities.

### Statistical analysis

Statistical analysis was performed using the SPSS statistical software (ref needed) for Chi square, 2 tailed Fisher exact test and Kaplan-Meier analysis. The results were judged significant at a confidence level greater than 95% (P ≤ 0.05).

### Example 2: Analysis of the expression of Δ133p53β in breast cancer patients

Primary, previously untreated operable breast cancer from 171 Caucasian women (age range 24-89 years; median age 64 years) with sufficient tumour tissue surplus to diagnostic requirements and complete clinical and pathological data were analysed.

### Results

In 171 breast cancers, expression of Δ133p53 in 50/171 (29%), Δ133p53β in 20/171 (11%) and Δ133p53γ in 27/171 (16%) was identified, whereas none of the three isoforms was detectable in normal breast tissue.

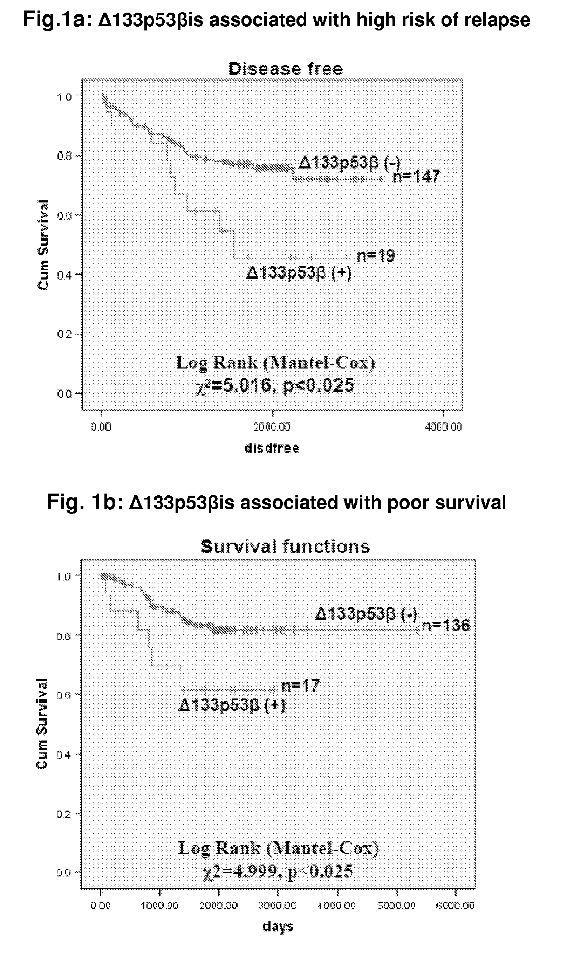

Δ133p53β expression was associated with axillary lymph node metastasis (Mann-Whitney analysis, exact 1-tailed, p<0.036) and in keeping with this, patients with tumours expressing Δ133p53β had a significantly worse disease free survival (log-rank, Cox-Mantel, p<0.025) (figure 1a) and overall survival (log-rank, Cox-Mantel, p<0.025) (figure 1b).

Δ133p53β was significantly associated with low ER expression (χ²=4.1, x d.f., p< 0.043 respectively) particularly for invasive ductal carcinoma (142/171 cases), the commonest histological type of breast cancer, (paired t-test, p<0.015).

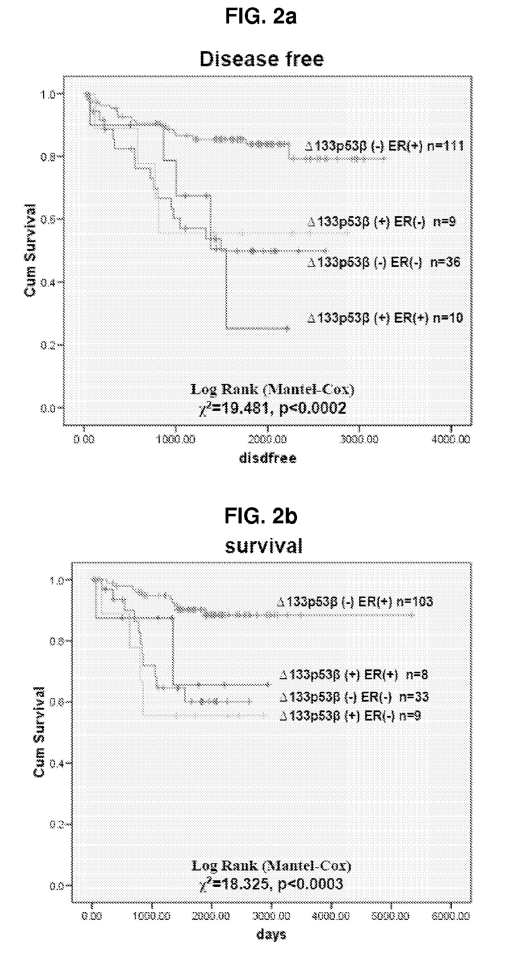

Figure 2a shows that ER positive patients expressing Δ133p53β had a significantly worse disease free survival than ER positive patients devoid of Δ133p53β expression (log rank, Cox-Mantel, p< 0.0002, pairwise comparison Δ133p53β+ ER+ vs Δ133p53β-ER+, p<0.003). Furthermore, figure 2b shows that ER positive patients expressing Δ133p53β had an unexpectedly poor survival comparable to ER negative patients (survival: log rank, Cox-Mantel, p< 0.0003, pairwise comparison Δ133p53β+ER+ vs ER-, p<0.658).

Δ133p53β was therefore associated with poor prognostic features (axillary node metastasis, ER negative,), shorter disease free survival and worse prognosis in primary breast cancer.

### Example 3: Main role of Δ133p53β isoform in the invasion process

First, the effects of the expression of GFP-tagged Δ133p53, Δ133p53γ and Δ133p53β isoforms on invasion of colon and breast carcinoma cells which retain epithelial characteristics and express wt p53 (hct116 for colorectal cancer and MCF7 for breast cancer) were tested, using invasion assays (see methods).

GFP alone transfected cells were used as negative controls. Cells were plated on top of a thick layer of matrigel which reproduces the physiological basal membrane. This *in vitro* assay aims to mimic the progress of tumour cells traversing basement membranes.

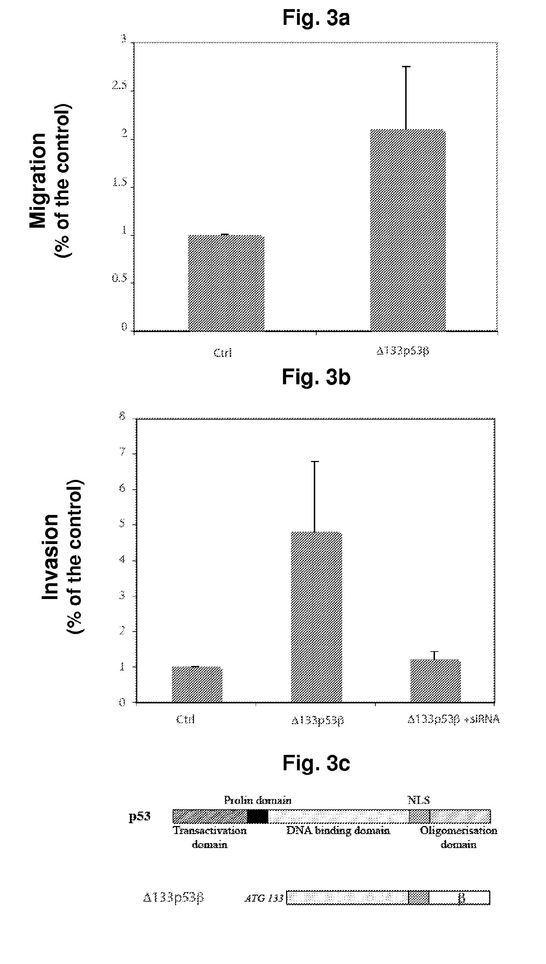

Figure 3a shows that invasiveness was significantly increased by the expression of the Δ133p53β isoform. Similar results were obtained with MCF7, (data not shown). This suggests that the overexpression of ectopic Δ133p53β isoform can overcome the anti-migratory activity of endogenous p53.

To determine which mode of migration was used by the cells expressing p53 isoforms to invade through the matrigel, the GFP-tagged isoforms expressing HCT 116 cells were filmed. Two populations of cells were observed: cohesive epithelial cells adherent to the glass and rounded cells which were above the cohesive ones, in an upper focus. Cells transfected with the GFP-tagged Δ133p53β isoform generated dynamic bleb-like structures on their surface. During the video, some rounded cells moved and that some cohesive adherent cells detached progressively from the epithelium and became rounded (data not shown). Loss of adhesive structures and concomitant acquirement of rounded-blebbing movement is strikingly similar to epithelial-amoeboid transition (EAT). A control by FACS that these blebbing movements were not due to apoptosis was realized (data not shown).

To quantify the penetrance of the phenotype, the rounded cells were separated from the cohesive cells and the GFP positive cells were counted in the two populations by FACS: for Δ133p53 and Δ133p53γ, the number of blebbing cells was 2.5 and 2.25 times more elevated than the number of adherent cells respectively, whereas for Δ133p53β, the number of blebbing cells was 4 times more elevated than the number of adherent cells (figure 4a). To conclude, this phenotype is significatively penetrant, i.e. cells expressing GFP-tagged Δ133p53β isoform adopt in large part a rounded morphology and the detachment of cells from the epithelia.

During EAT, tight junctions are first dissociated, then adherent junctions disappear afterwards. Since E-cadherin is the principal component of adherent junctions, it is widely used as a specific marker of the integrity of these junctions. In p53 expressing cells, E-cadherin was expressed at high level in adherent cohesive cells whereas E-cadherin expression was lost in blebbing cells (figure 4b), confirming that blebbing cells have lost a key epithelial feature.

Tumour cells having undergone EAT express low levels of beta 1-integrin and so adopt amoeboid-like migration independent of the integrin-adhesion process. The expression of the beta 1-integrin in hct116 cells expressing Δ133p53, Δ133p53γ and Δ133p53β isoform was evaluated. As for E-cadherin, blebbing cells did not express beta 1-integrin. (figure 4c). The same results were obtained using myc- or GFP-tagged isoforms. Expression of the myc-tagged isoforms was controlled by western blot using an anti-myc antibody (data not shown).

These results show that expression of N-terminus deleted p53 isoforms promotes epithelial-amoeboid transition in hct116 cells, which consequently migrate through rounded blebbing movements. This mode of migration is more effective than mesenchymal migration indicating that expression of these Δ133p53β variants in tumours could be associated with a poor prognosis for patients. This reinforces the idea that failure of appropriate regulation of the p53 isoform expression may have dramatic consequences on tumour progression and metastasis.

Since rounded blebbing-associated mode of motility is dependent on ROCK (Rho Kinase) signalling, invasion assays in the presence of the ROCK inhibitor, Y27632 were performed. The protocol was as described above.

The results show that the treatment with Y27632 highly reduced the invasiveness of transfected cells indicating that ROCK activity is required for invasiveness provided by Δ133p53 isoforms expression.

Recently, it has been reported that the two homologous ROCKs may not be functionally equivalent. To determine whether both ROCK isoforms have identical effects on the p53 isoforms-dependent rounded blebbing associated movements, the expression of ROCK I and ROCK II in transfected cells was studied. The results show that the expression of ROCK I was increased in adherent cells (as compared with control cells, T) but was importantly decreased in blebbing cells. Conversely, ROCK II was expressed at high levels in all situations, and was in particular maintained in blebbing cells. This suggests that ROCK II is preferentially required for blebbing movement whereas ROCK I may be involved in the first steps of the epithelial-amoeboid transition. Despite their high sequence homology (65% overall identity and 92% identity in their kinase domain) ROCK I and ROCK II do not function in a redundant manner: there is no compensation for the loss of ROCK II by ROCK I in knock out mice and the two isoforms regulate different aspects of myosin II activity. It was thus shown for the first time different implications of the two ROCK isoforms in amoeboid-like migration and consequently in invasiveness.

ROCK-driven actin reorganisation during rounded-blebbing motility is largely dependent on the Rho GTPase family protein RhoA.

To investigate the mechanism by which p53 isoforms exert their effects on ROCK signalling, the expression of RhoA in adherent or blebbing cells expressing the three Δ133 isoforms of p53 was examined.

Total levels of RhoA were identical when cells did or did not express the p53 Δ133 isoforms (data not shown). This expression was maintained in blebbing cells. Nevertheless, the level of expression of the protein does not reflect kinase activity. RhoA activity in adherent versus blebbing cells expressing, or not, the Δ133p53 isoforms were compared using pull-down assays that capture only the active GTP-bound form of RhoA. First, we observed a basal activity of RhoA in adherent epithelial cells. However, this activity was highly increased in blebbing cells. Interestingly, the increase obtained with Δ133β-p53 was 2.5-3 fold higher than those obtained with the others isoforms, Δ133-p53 and Δ133γ-p53 which correlated with the important capability of Δ133β-p53 expressing cells to lose surface adhesion.

The question arises as to whether the activation of RhoA in Δ133-p53 isoform expressing cells was due to an overexpression of Guanine Exchange activating Factors (GEFs). The study was focused on two GEFs of RhoA which have both been involved in mediating the p53-dependent regulation of RhoA: GEF-H1, which has been found to be activated by mutant p53 and which expression is strongly correlated with p53 status in cancer cells and ECT2 which is known to be a transcriptional target of wt p53 and which is involved in the regulation of epithelial junctions.

It was found that GEF-H1 was overexpressed in blebbing cells expressing the three isoforms of p53. By contrast, the expression of ECT2 decreased in blebbing cells, as compared with adherent cells. This down-regulation of ECT2 in blebbing cells is compatible with its known function as a regulator of epithelial junctions, but does not take account for the RhoA activation, which may depend on the up-regulation of GEF-H1. This indicates distinct roles for GEF-H1 and ECT2 during the epithelio-amoeboid transition. The results are consistent with the transformation ability of GEF-H1 when transfected in mouse fibroblasts and the induction of tumour when cells transfected with GEF-H1 are injected in nude mice. Two opposing pathways downstream of RhoA are working on the regulation of adherent junctions: a ROCK-dependent pathway involved in the disruption of epithelial junctions and a Dia-dependent route promoting the formation of cadherin-catenin complexes and stabilisation of junctions. One possibility is that these two pathways are activated by two different GEFs for RhoA: ECT2 could activate the RhoA-Dia pathway to promote adherent junction as this GEF regulates epithelial polarity and GEF-H1 could activate the RhoA-ROCK pathway to disrupt cell-cell junction and promote epithelial-amoeboid transition. p53 is the upstream mediator of these two pathways and expression of Δ133 isoforms of p53 deregulate the balance between the two pathways during tumorigenesis.

The data show that dysregulated expression of Δ133p53 isoforms (isoforms deleted in the N-terminal domain) confers increased motility and invasiveness to carcinoma cells. This indicates that changing the ratio of the p53 isoforms favour increased tumour aggressiveness and enhanced metastatic capability for cancer cells, and highlights the importance of splicing events during cancer progression.

## Claims

1. A method of testing a subject thought to be predisposed to having cancer which comprises the step of:
i) analyzing a biological sample from said subject for detecting the presence, the expression level or the absence of a p53 isoform selected in the group consisting in Δ133p53, Δ133p53γ and Δ133p53β, the presence of said isoform being indicative of cancer.

2. The method according to claim 1, including a further step of comparing the detected expression level of the p53 isoform with a positive or a negative control.

3. A method for measuring the aggressiveness of cancer in a subject, which comprises the step of:
i) determining the presence, the expression level or the absence of a p53 isoform selected in the group consisting in Δ133p53, Δ133p53γ and Δ133p53β in a biological sample from the subject, the presence of said isoform being indicative of an aggressive cancer, preferably said isoform being indicative of a metastatic cancer.

4. A method for determining the subject's response to an anti-cancer therapy, said subject is receiving or has received therapy for a state associated with cancer, which comprises the step of:
i) determining the expression level of a p53 isoform selected in the group consisting in Δ133p53, Δ133p53γ and Δ133p53β in a biological sample from the subj ect and
ii) comparing it to a control.

5. The method according to any of the previous claims, wherein the p53 isoform is Δ133p53β.

6. The method according to any of the previous claims, wherein the step of detecting the presence, the expression level or the absence of a p53 isoform of p53 isoforms is be evaluated by detecting a p53 isoform polypeptide or a fragment thereof, or by detecting a p53 isoform mRNA or a fragment thereof.

7. The method according to any of the previous claims, comprising a further step of amplifying said p53 isoform mRNA or cDNA, the complementary sequence thereof, or a fragment thereof having at least 10 nucleotides length which is specific of said p53 isoform.

8. The method according to any of the previous claims, wherein the determination of the presence or the absence of a p53 isoform selected in the group consisting in Δ133p53, Δ133p53γ and Δ133p53β, is carried out by a probe capable of specifically hybridizing with said p53 isoform mRNA, complementary sequence thereof or a fragment thereof having at least 10, 15, 20, 25, 30, 35, 40, 45, 50 or more nucleotides length which is specific of said p53 isoform, preferably hybridizing with Δ133p53β mRNA having the sequence SEQ ID NO: 2.

9. The method according to 1 to 7, wherein the determination of the presence, the expression level or the absence of mRNA of a p53 isoform selected in the group consisting in Δ133p53, Δ133p53γ and Δ133p53β, is carried out by the following method comprising the steps of:
(i) contacting a nucleic acid probe specific of said p53 isoform under hybridizing conditions with the biological test sample comprising either;
(ii) RNA molecules isolated from a biological sample of the human subject, wherein the biological sample is suspected of containing tumour cells, or (ii) nucleic acid molecules synthesized from the isolated RNA molecules as cDNA;
wherein the nucleic acid probe has a nucleotide sequence comprising either a fragment of at least 15 nucleotides length of the sequence of the p53 isoform, or a fragment thereof, or their complement, and
(b) detecting the formation of hybrids of the nucleic acid probe and the test sample;
wherein the presence of hybrids indicates the presence of tumour cells in the tissue obtained from the human subject.

10. The method according to any of claims 1 to 7, wherein the determination of the presence or the absence of a p53 isoform selected in the group consisting in Δ133p53, Δ133p53γ and Δ133p53β, is carried out by contacting the biological sample with an antibody specific of the p53 isoform polypeptide or a fragment thereof and determining the binding of the antibody to the biological sample.

11. A method of screening potential anti-cancer compounds, which comprises the steps of:
a) optionally measuring in a cell known to express a p53 isoform selected in the group consisting in Δ133p53, Δ133p53γ and Δ133p53β, the expression level of said p53 isoform;
b) contacting the compound to be tested with said cell;
c) determining the expression level of said p53 isoform by a method of detecting said p53 isoform according to claims 1 to 10; and
d) selecting said compound as a potential anti-cancer compound if the p53 isoform is not expressed in the cell, or has an expression level lower than before step a).

12. A method according to any of the previous claims, wherein the cancer is a breast cancer or a colon cancer.

13. A method according to any of the previous claims, wherein the cancer is a metastatic cancer.

14. A method for the production of polyclonal antibodies specifically recognizing a p53 isoform polypeptide selected in the group consisting in Δ133p53, Δ133p53γ and Δ133p53β polypeptide, wherein such method comprises the step of:
a) immunization of a mammal animal with a immunologically effective amount of a p53 isoform polypeptide selected in the group consisting in Δ133p53, Δ133p53γ and Δ133p53β, or against an specific epitope fragment of at least 9 amino acids length of said polypeptide, optionally with an enhancing carrier preparation;
b) optionally, *in vitro* determining the presence of specific antibodies in the animal serum or plasma; and
c) purifying or isolating the specific anti- Δ133p53, anti- Δ133p53γ or anti-Δ133p53β polypeptide from the animal serum or plasma.

15. A method for the production of a hybridoma cell capable of secreting monoclonal antibodies specifically recognizing a p53 isoform selected in the group consisting in Δ133p53, Δ133p53γ and Δ133p53β polypeptides, wherein such method comprises the step of:
a) immunization of a mammal animal with a immunologically effective amount of a p53 isoform selected in the group consisting in Δ133p53, Δ133p53γ and Δ133p53β, or against an epitope fragment of at least 9 amino acids length of said polypeptide, optionally with an enhancing carrier preparation;
b) isolating antibodies anti- p53 isoform peptide producing lymphocytes from the spleen, lymph nodes or peripheral blood of that mammal animal; and
c) immortalizing the antibodies anti-Δ133p53, anti- Δ133p53γ or anti-Δ133p53β polypeptide producing lymphocytes by fusion of said lymphocytes to cells of same species mammal animal myeloma line.

16. A method for the production of monoclonal antibodies specifically recognizing a p53 isoform selected in the group consisting in Δ133p53, Δ133p53γ and Δ133p53β polypeptide, wherein such method comprises the step of:
a) producing an hybridoma cell capable of secreting monoclonal antibodies specifically recognizing a p53 isoform selected in the group consisting in Δ133p53, Δ133p53γ and Δ133p53β polypeptides, according to claim 15;
b) culturing such hybridoma cell in appropriate culture medium and culture conditions;
c) purifying or isolating from such culture medium the monoclonal antibodies which are secreted.

17. Kit for the detection or quantification of a p53 isoform selected in the group consisting in Δ133p53, Δ133p53γ and Δ133p53β mRNA or polypeptide, wherein such kit comprises:
a) a polyclonal or monoclonal antibody specifically recognizing said p53 isoform; or
b) a pair of primers selecting from the group consisting of a pair of primers capable of amplifying the sequence SEQ ID NO: 2, SEQ ID NO: 4 or SEQ ID NO: 6, or a fragment thereof having at least 10 nucleotides length; or
c) a probe having a nucleotide sequence comprising either a fragment of at least 10 nucleotides length of a sequence of p53 isoform selected in the group consisting in Δ133p53, Δ133p53γ and Δ133p53β, or their complement.
